# EUROPEAN PATENT APPLICATION

(11) **EP 0 535 587 A1**
(43) Date of publication of application: **07.04.1993**
(21) Application number: 92116632.8
(22) Date of filing: 29.09.1992
(51) Int. Cl.: C12Q 1/68, G01N 21/64, G01N 27/447

(54) **Process for optimizing nucleotide sequence determination**

(30) Priority: 30.09.1991 US 768491
(71) Applicant: BECKMAN INSTRUMENTS, INC., Fullerton California 92634-3100 (US)
(72) Inventor: Konrad, Kenneth D., Long Beach, California 90814 (US); Pentoney, Stephen L., Jr., Yorba Linda, California 92686 (US)
(74) Representative: Kahler, Kurt, Dipl.-Ing.

(57) **Abstract**

Disclosed herein is a process for optimizing nucleotide sequence determination based upon the dideoxynucleotide triphospate chain determination protocol, where at least three of the four ddNTPs define a concentration ratio arrangement and are used to determine a first nucleotide sequence, the concentration of each ddNTP being different than the others, and at least one subsequent nucleotide sequence is determined using at least three of the four ddNTPs in the same defined concentration ratio arrangement, whereby different ddNTPs occupy the positional order of said concentration ratio arrangement in said first and subsequent nucleotide sequence determinations.

## Description

This application is related to a copending EP application claiming the priority of US Serial No. 07/769,422, filed September 30, 1991, entitled "NUCLEOTIDE SEQUENCE DETERMINATION EMPLOYING MATCHED DIDEOXYNUCLEOTIDE TERMINATOR CONCENTRATIONS" by the same inventors. A copy of this application has been submitted for filing together with the present application.

The present invention is generally directed to processes for determination of the sequence of nucleotides, and in particular, to a new method for optimizing the determination of the base sequence of either deoxyribonucleic acid ("DNA") or ribonucleic acid ("RNA") based upon the dideoxynucleotide triphosphate ("ddNTP") chain termination method.

References set forth in the Background of the Invention are incorporated herein by reference.

DNA and RNA are long, threadlike macromolecules, DNA comprising a chain of deoxyribonucleotides, and RNA comprising a chain of ribonucleotides. A nucleotide consists of a nucleoside and one or more phosphate groups; a nucleoside consists of a nitrogenous base linked to a pentose sugar. Typically, the phosphate group is attached to the fifth-carbon ("C-5") hydroxyl group ("OH") of the pentose sugar. Accordingly, such compounds are typically referred to as nucleoside 5'-triphosphates or 5'-nucleotides.

In a molecule of DNA, the pentose sugar is deoxyribose, while in a molecule of RNA, the pentose sugar is ribose. The nitrogenous bases in DNA can be adenine ("A"), cytosine ("C"). guanine ("G"), or thymine ("T"). These bases are the same for RNA, except that uracil ("U") replaces thymine. Accordingly, the major nucleotides of DNA, collectively referred to as "deoxynucleotide triphosphates" ("dNTPs"), are as follows: deoxyadenosine 5'-triphosphate ("dATP"); deoxycytidine 5'-triphosphate ("dCTP"); deoxyguanosine 5'-triphosphate ("dGTP"); and deoxythymidine 5'-triphosphate ("dTTP"). The major nucleotides of RNA are as follows: adenosine 5'-triphosphate ("ATP"); cytidine 5'-triphosphate ("CTP"); guanosine 5'-triphosphate ("GTP"); and uridine 5'-triphosphate ("UTP").

The sequence of the nitrogenous bases of the DNA or RNA molecule encodes the genetic information contained in the molecule. The sugar and phosphate groups of a DNA or RNA molecule perform a structural role, forming the backbone of the molecule. Specifically, the sugar moiety of each nucleotide is linked to the sugar moiety of the adjacent nucleotide such that the 3'-hydroxyl of the pentose sugar of one nucleotide is linked to the 5'-hydroxyl of the pentose sugar of the adjacent nucleotide. The linkage between the two pentose sugars is via a phosphodiester bond. Based upon this linkage protocol, one end ("terminus") of the nucleotide chain has a 5'-terminus (e.g. hydroxyl, triphosphate, etc.), and the other end has a 3'-hydroxyl group. By convention, the base sequence of nucleotide chains is written in a 5' to 3' direction, i.e., 5'-ATCG-3', or simply ATCG.

The formation of the phosphodiester bond between deoxyribonucleotides is brought-about by the enzyme DNA polymerase. In order for DNA polymerase to synthesize a chain of DNA, the following components are required: (1) a single stranded DNA molecule, referred to as a "template"; (2) a short DNA strand, having a free 3'-hydroxyl group, which is hybridized to a specific site on the template, the short strand being referred to as a "primer"; and (3) dNTPs. Elongation of the primer proceeds in the 5' to 3' direction. DNA polymerase brings-about the formation of a phosphodiester bond most typically when the base of the incoming nucleotide is complementary to the base of the nucleotide on the template and only if the base of the nucleotide on the primer has a 3'-hydroxyl group.

Two complementary chains of nucleotides, held together by (relatively) weak hydrogen bonds between the nucleotides, form a complete DNA or RNA macromolecule. The specificity of binding between the bases is such that A always binds to T (or U in the case of RNA), and C always bonds with G. Thus, for the sequence 5'-ATCG-3', the sequence 3'-TAGC-5' will lie immediately across therefrom. Because of this specificity in binding, the sequence of a single-stranded template of DNA or RNA can be determined by determining the bases which bind to the template. This, in essence, is the basis for nucleotide sequencing.

Another unique and useful form of NTPs exist. These are referred to as chain terminating dideoxynucleotide triphosphates, or "ddNTPs." ddNTPs differ from dNTPS in that they lack a 3'-hydroxyl group. Accordingly, while ddNTPs can be incorporated into the growing primer strand via the 5'-triphosphate portion thereof, the absence of a 3'-hydroxyl group prevents formation of a phosphodiester bond with a succeeding dNTP (or ddNTP). Accordingly, once a ddNTP is incorporated into the primer strand, further extension of that strand is not possible.

DNA sequencing protocols, particularly those suited for automated DNA sequencing instrumentation formats, principally rely upon the methodology developed by Sanger et al., Proc. Natl. Acad. Sci. USA 74:5463-5467 (1977) (hereinafter, "Sanger et al."). Generally, the Sanger et al. protocol involves four separate syntheses, whereby a single stranded template (obtained via, e.g., denaturation of double-stranded DNA or cloning of the DNA template into, e.g., bacteriophage M13 vector), is provided with a primer such that elongation of the primer, via DNA polymerase, proceeds. Each reaction is terminated (via the appropriate ddNTP utilized in that reaction) at one of the four bases, i.e., A, T, C, or G, via the incorporation of the appropriate chain terminating agent. Thus, if the templates have the sequence 5'-XXXATGCTGCA-3' and the primer is complementary to XXX, the addition of dGTP, dCTP, dTTP, dATP and ddATP, as well as DNA polymerase, would lead to the formation of two primer-extension fragments: 5'-XXXTA-3' and 5'-XXXTACGA-3', which are complementary to the template. In a second synthesis, the protocol would be the same, except that, e.g., ddTTP would be utilized instead of ddATP, leading to the formation of 5'-XXXT-3' and 5'-XXXTACGACGT-3'. The third synthesis would utilize ddCTP (5'-XXXTAC-3' and 5'-XXXTACGAC-3'), and the fourth would utilize ddGTP (5'-XXXTACG-3' and 5'-XXXTACGACG-3'). By utilizing a labelled primer, ddNTPs or dNTPs, and subjecting the various extension products to gel electrophoresis, various discrete bands will be obtained on the resulting gel, due to the various electrophoretic mobilities of the extension fragments. From these bands, one can determine the sequence of the extension fragments, such that the sequence of the template is readily determined therefrom.

Using the above methodology, automated systems for nucleotide sequence analysis have been developed.

Smith, et al., 321 Nature 674-678 (1986), describe the use of four primers, each labelled with a different fluorescent marker. Each primer is used in a separate reaction mixture containing one of the four ddNTPs, followed by combining the reaction mixtures. This combined mixture is then electrophoresed down a single lane on a gel, and a laser is utilized to detect the fluorescent products, such that the sequence can be determined.

Prober, et al., 238 Science 336-341 (1987), describe the use of the four ddNTPs, each having a different label, whereby a single polymerase reaction takes place followed by single-lane electrophoresis. Detection of the different labels is then made in an effort to determine the sequence.

Toneguzza, et al., 6 BioTechniques 460-469 (1988), describe the use of radioactive labelled primer in conjunction with four separate syntheses using the four ddNTPs, followed by electrophoresis in four separate lanes. Analysis of the radioactive bands is then used to determine sequence information. Kambara, et al., 6 BioTechniques 816-821 (1988) utilize a fluorescent labelled primer in place of the radioactively labelled primer of Toneguzza, et al.

Ansorge, et al., 20 J.Biochem. Biophys. Meth. 47-52 (1989), describe a single-label primer, two-lane chain terminating method, whereby in one lane the concentration of one ddNTP is significantly higher than that of a second ddNTP, and in the second lane this ratio is repeated for the remaining two ddNTPs. Thus, the results of each lane are "superimposed such that the complete sequence can be determined.

Tabor and Richardson, 265 J. Biochem. 8322-8328 (1990) describe a protocol whereby a genetically modified version of T7 DNA polymerase, in conjunction with manganese (to reduce the discrimination between incorporation of dNTPs versus ddNTPs) are utilized with fluorescently labeled primers using four different concentrations of the ddNTPs; following electrophoresis in a single lane, the sequence is determined via the relative intensity of each fragment. See also European Application Number 89306877.5 (publication number A2 0351138) ("hereinafter "Tabor-Richardson"), and, Tabor and Richardson 86 Proc. Natl. Acad. Sci. USA 4076-4080 (1989).

Tabor-Richardson summarizes certain of the problems associated with previous protocols for nucleotide sequence analysis, for example, differences or variations in signal intensities between sequencing bands, and describes a protocol to correct these problems. The described approach offers at least three advantages over the previous methods: (1) the protocol relies upon a single reaction, which simplifies the chemistry; (2) the protocol relies upon a single label, which increases the efficiency of detection; and (3) the protocol relies upon a single "lane", which maximizes throughput.

A first drawback to the Tabor-Richardson protocol is that it is dependent upon the performance of the polymerase, i.e., the ability of the polymerase in not discriminating between incorporation of dNTPs and ddNTPs onto the growing primer strand, or, the ability of the polymerase to uniformly incorporate ddNTPs into the growing primer strand that accurately reflect the concentration of the ddNTPs being utilized. Stated again, the protocol is susceptible to non-uniform ddNTP incorporation by the polymerase. A second drawback to the protocol is that it is susceptible to compression artifacts occasioned by, e.g., "shoulder peaks," whereby two different nucleotides appear as a semi-singular peak, are difficult to call using the foregoing protocol.

These deficiencies can lead to erroneous calls for nucleotides within the template sequence, thus leading to skepticism as to the integrity of the entire sequence determination.

What is needed, then, is a process for sequencing nucleotides which advantageously utilizes the dideoxynucleotide triphosphate chain termination protocol and ensures that a putative sequence determined by the protocol is the correct sequence.

The present invention satisfies this and other needs. This is preferably accomplished by providing a process for sequencing a strand of nucleotides using the dideoxynucleotide triphosphate chain terminating method, the process comprising the steps of obtaining a first data set nucleotide sequence using, inter alia, the four dideoxynucleotide triphosphate chain terminators in a concentration ratio of [ddNTP₁] ("first position") > [ddNTP₂] ("second position") > [ddNTP₃] ("third position") > [ddNTP₄] ("fourth position"), and thereafter obtaining at least one additional data set nucleotide sequence using, inter alia, the chain terminators in a concentration ratio different from the first data set concentration ratio, the additional data set chain terminator concentration ratio being selected from the group consisting of:
(a) [ddNTP₄] > [ddNTP₃] > [ddNTP₂] > [ddNTP₁];
(b) [ddNTP₄] > [ddNTP₃] > [ddNTP₁] > [ddNTP₂];
(c) [ddNTP₄] > [ddNTP₂] > [ddNTP₁] > [ddNTP₃];
(d) [ddNTP₄] > [ddNTP₂] > [ddNTP₃] > [ddNTP₁];
(e) [ddNTP₃] > [ddNTP₄] > [ddNTP₂] > [ddNTP₁];
(f) [ddNTP₃] > [ddNTP₄] > [ddNTP₁] > [ddNTP₂];
(g) [ddNTP₃] > [ddNTP₁] > [ddNTP₄] > [ddNTP₂];
(h) [ddNTP₃] > [ddNTP₁] > [ddNTP₂] > [ddNTP₄];
(i) [ddNTP₂] > [ddNTP₄] > [ddNTP₃] > [ddNTP₁];
(j) [ddNTP₂] > [ddNTP₄] > [ddNTP₁] > [ddNTP₃];
(k) [ddNTP₂] > [ddNTP₁] > [ddNTP₄] > [ddNTP₃]; and
(l) [ddNTP₂] > [ddNTP₁] > [ddNTP₃] > [ddNTP₄],
where each numerical subscript 1-4, inclusive, represents one of the four bases, A, C, G, and T (or U in the case of RNA). Comparative analysis of both data sets is preferably conducted whereby an accurate nucleotide sequence is defined.

In a preferred embodiment, the first data set is determined whereby [ddNTP₄] is substantially zero for the first data set only, and the concentration of the fourth position ddNTP is substantially zero for subsequent data set determinations. By "substantially zero" is meant that the concentration of ddNTP₄ in the first data set determination and the concentration of the fourth position ddNTP in subsequent data sets is no greater than about 25%, preferably no greater than about 15%, and most preferably no greater than about 5% of the concentration of ddNTP₃ used for the first data set determination, and of the concentration of the third position ddNTP in subsequent data set determinations.

In a particularly preferred embodiment, the first data set is determined whereby [ddNTP₄] is substantially zero for the first data set only, and the complementary second data set is determined using the chain terminators in a concentration ratio selected from the group consisting of:
(a) [ddNTP₃] > [ddNTP₄] > [ddNTP₂] > [ddNTP₁];
(b) [ddNTP₃] > [ddNTP₄] > [ddNTP₁] > [ddNTP₂];
(c) [ddNTP₂] > [ddNTP₄] > [ddNTP₃] > [ddNTP₁]; and
(d) [ddNTP₂] > [ddNTP₄] > [ddNTP₁] > [ddNTP₃],
whereby for the second data set determination, the fourth position is substantially zero, i.e., in groups (a) and (c) the concentration of ddNTP₁ is substantially zero, in group (b) the concentration of ddNTP₂ is substantially zero, and in group (d) the concentration of ddNTP₃ is substantially zero.

In its most preferred embodiment, the concentration values for the first position, second position, third position and fourth position ddNTPs for the first data set are substantially equivalent to the concentration values for the first position, second position, third position and fourth position ddNTPs of the second data set, and the concentration ratio for the chain terminators for the second data set is selected from the group consisting of:
(a) [ddNTP₃] > [ddNTP₄] > [ddNTP₂] > [ddNTP₁]; and
(b) [ddNTP₃] > [ddNTP₄] > [ddNTP₁] > [ddNTP₂].
For example, if the concentration ratios of the ddNTPs for the first data set determination are, e.g., 4:2:1:0, then the concentration ratios of the ddNTPs for the second data set determination are 4:2:1:0, irrespective of the ddNTPs occupying the first position, second position, third position or fourth position for the second data set determination. As used herein, the term "substantially equivalent" means plus or minus about 10% (i.e., values between 4.4 and 3.6 are substantially equivalent to 4).

By re-arranging the concentration order of the chain terminators, the data set sequences will be substantially the same, but the resulting signals (e.g., band intensity, peak heights, peak areas) will be different between the data sets, based upon the concentrations of the respective ddNTPs. Accordingly, close calls and artifacts can be rapidly and efficiently distinguished. Therefore, by comparing the first data set nucleotide sequence with the second data set nucleotide sequence, the accuracy and resolution of the actual nucleotide sequence is significantly enhanced.

### BRIEF DESCRIPTION OF THE DRAWINGS

FIG. 1 is an idealized electropherogram representation of the sequence ACTG where the concentration ratio of ddATP:ddCTP:ddTTP:ddGTP is 4:2:1:0;
FIG. 2 is an idealized electropherogram representation of the sequence ATCG where the concentration ratio of ddTTP:ddGTP:ddCTP:ddATP is 4:2:1:0;
FIG. 3 is FIG. 2 (dashed lines) superimposed upon FIG. 1 (solid lines);
FIG. 4 is an electropherogram of the results of the analysis of a portion of the DNA sequence of the bacteriophage M13mp18 having as an actual sequence: 
5'-TTCCACACAACATACGAGCCGGAAGCATAAAG
TGTAAAGCCTGGGGTGCCTAATGAGTGAGCTAA-3'
 where the dideoxynucleotides ddCTP, ddTTP, ddGTP and ddATP were present in a concentration ratio of 4:1.7:0.7:0;
FIG. 5 is an electropherogram of the same portion of the M13mp18 DNA of FIG. 4 where the dideoxynucleotides ddATP, ddGTP, ddCTP and ddTTP were present in a concentration ratio of 4:1.7:0.7:0;
FIG. 6 is superimposed electropherograms of the results of the analysis of a portion of the DNA sequence of the bacteriophage M13mp18 having as an actual sequence: 
5′-CGAATTCGTAATCATGGTC
ATAGCTGTTTCCTGTGTGA-3′
 where the dideoxynucleotides ddTTP, ddCTP, ddGTP and ddATP were present in a concentration ratio of 4:2:1:0 (dashed lines), and the dideoxynucleotides ddATP, ddGTP, ddCTP and ddTTP were present in a concentration ratio of 4:2:1:0 (solid lines); and
FIG. 7 is an electropherogram of the same portion of the M13mp18 DNA of FIG. 6 where the dideoxynucleotides ddCTP, ddTTP, ddGTP and ddATP were present in a concentration ratio of 4:2:1:0.

For purposes of brevity, the following portion of the disclosure focuses on single stranded DNA analysis. Those skilled in the art are credited with the ability to readily adapt the following to double stranded DNA. Double stranded DNA, for example, is preferably separated prior to analysis by any strand separation means available in the art, including, but not limited to, heat denaturation, enzymatic denaturation, or chemical denaturation. Additionally, and again for purposes of brevity, the following focuses on high performance capillary electrophoresis as a means for separating DNA fragments according to molecular weight. The skilled artisan is also credited with the ability to readily adapt the following to other DNA separation protocols, including, but not limited to, gel electrophoresis.

As used herein, the term "label" includes, but is not limited to, radioisotopic labels and fluorescent labels. Of these, fluorescent labels are preferred due to the ease of handling and costs associated therewith. Representative radioisotopic labels include, e.g., ³²P and ¹²⁵I, although any radioactive label capable of providing sufficient detection sensitivity and which does not significantly impair the hybridization of the primer to the template (in cases where the primer is labelled), or the incorporation of the dNTPs or ddNTPs onto the growing primer strand (in cases where the dNTPs or ddNTPs, respectively, are labelled), is applicable. While not exhaustive or limiting, the following criteria can be utilized in selecting a fluorescent label: (1) the absorption and emission maxima are preferably in the visible range of the spectrum, preferably as far to the red end as possible; (2) the label has significant quantum efficiency to provide sufficient detection sensitivity; and (3) the label should not significantly impair the hybridization of the primer to the template (in cases where the primer is labelled), or the incorporation of the dNTPs or ddNTPs onto the growing primer strand (in cases where the dNTPs or ddNTPs, respectively, are labelled). Representative fluorescent dyes include, but are not limited to, fluorescein, fluorescein derivatives (such as, for example, succinylfluorescein dyes such as those described in Prober, et al., and fluorescein isothiocyanate as described in Kambara, et al.), NBD aminohexanoic acid, rhodamine and rhodamine derivatives, such as, for example, Texas Red™ (Molecular Probes, Junction City, Oregon), tetramethylrhodamine isothiocyanate (Research Organics, Inc., Cleveland, Ohio), as described in Smith et al, and BODIPY™ and BODIPY™-derivatives (Molecular Probes). A preferred label is a BODIPY™-derivative having an excitation wavelength of about 543nm and an emission wavelength of about 580nm.

As used herein, agents which are capable of bringing about the formation of a phosphodiester bond between nucleotides are referred to as "catalytic enzymes" or "polymerases." Polymerases which are applicable to the present invention include, but are not limited to, the large fragment of Escherichia coli DNA polymerase I ("Kenlow" fragment), Taq polymerase, reverse transcriptase, T7 DNA polymerase, and modified forms of T7 DNA polymerase as described in Tabor & Richardson, 1989. Particularly preferred catalytic enzymes include DNA polymerases belonging to a class of homologous polymerases including T7-type DNA polymerases (e.g., T7, T3, ΦI, ΦII, H, W31, gh-I, Y, AII22 and SP6), "Kenlow" fragment, and Taq polymerase. As used herein, the term "homologous polymerases" means a catalytic enzyme that discriminates against ddNTPs compared to dNTPs in the presence of magnesium, with reduced discrimination when magnesium is replaced by manganese, or a catalytic enzyme that naturally evidences a reduced discrimination between dNTPs and ddNTPs. A particularly preferred catalytic enzyme is available under the brand name Sequenase™ (US Biochemicals, product no. 70775; the kit also includes therein manganese buffer).

As used herein, the term "chain terminating agent" means an agent: that is capable of specifically terminating a nucleotide sequencing reaction at a specific nucleotide type; that can be incorporated into a growing primer strand; and that can properly bind to a nucleotide on the template. Preferred chain terminating agents include dideoxyribonucleotides, and in particular, dideoxyribonucleotides having a 2',3' dideoxy structure. It is noted that the protocol disclosed herein allows for the use of labelled chain terminating agents. However, as those in the art appreciate, the label portion of a labelled chain terminating agent can lead to discrimination by the catalytic enzyme, and thus an inability to be incorporated into the growing primer strand. In such a case, a preferred approach is to utilize a labelled primer in conjunction with unlabelled chain terminating agents, or to utilize a different label which does not lead to such discrimination. Those skilled in the art are credited with the ability to readily optimize these conditions in conjunction with the particular needs, objectives and desires of the artisan.

The disclosed optimization protocol finds particular applicability in any nucleotide sequencing protocol that relies upon the so-called dideoxynucleotide chain termination protocol, irrespective of: the type of nucleotide chain (DNA or RNA); whether the primer, the dNTPs, or the ddNTPs are labelled; the type of catalytic enzyme utilized; or, whether the analysis is conducted in a single "lane" (whereby the ddNTPs are utilized in conjunction with at least two labels) or multiple "lanes" (whereby the ddNTPs are utilized in conjunction with at least one label).

A dideoxynucleotide chain termination protocol for sequencing a strand of DNA can comprise the following steps (although, in view of the foregoing, other protocols can utilize variations on these steps, or different steps, while still relying upon the dideoxynucleotide chain termination protocol):
(a) providing a template strand of DNA;
(b) annealing the strand with a labelled primer capable of hybridizing to the strand to give an annealed mixture;
(c) incubating the annealed mixture with: the four dNTPs, each dNTP having approximately the same concentration as the others; the four ddNTPs, where the concentrations of each ddNTP is different than the others; and DNA polymerase, under conditions in which the DNA polymerase causes the production of a series of four different DNA products, each product having the same labeled primer at a first terminus, and one of the four ddNTPs at a second terminus;
(d) separating the series of four different DNA products from the strands of DNA;
(e) separating the series of four different DNA products from each other according to molecular weight to form an arrangement of four different signals, each signal being specific to the concentration of each of the four ddNTPs; and
(f) determining the position of each of the four ddNTPs based upon the location of the four signals in the arrangement.
It is in the context of the foregoing protocol that the present protocol for optimizing the determination of a nucleotide sequence is made manifest.

In accordance with the disclosure herein, a process for optimizing the determination of a nucleotide sequence comprises the steps of:
(a) providing a template strand of DNA;
(b) annealing the template with a primer having attached thereto a first label, said first labelled primer capable of hybridizing to the template to give an annealed mixture;
(c) incubating the annealed mixture with: the four dNTPs, each dNTP having approximately the same concentration as the others; the four ddNTPs, said ddNTPs being present in a concentration ratio [ddNTP₁] > [ddNTP₂] > [ddNTP₃] > [ddNTP₄] where each numerical subscript 1-4, inclusive, represents one of the four bases, A, C, G, and T and where [ddNTP₄] is substantially zero; and a first DNA polymerase, under conditions in which the first DNA polymerase causes the production of a first series of at least three different DNA products, each product having the same labeled primer at a first terminus, and a ddNTP at a second terminus;
(d) separating the first series of different DNA products from the template;
(e) separating the first series of different DNA products from each other according to molecular weight to form a first arrangement of at least three different signals, each signal being specific to the concentration of ddNTPs;
(f) obtaining a first data set nucleotide sequence by determining the position of each of the four ddNTPs based upon the location of the signals in the first arrangement;
(g) providing said template;
(h) annealing the strand with a primer having attached thereto a second label, said labelled primer capable of hybridizing to the template to give an annealed mixture;
(i) incubating the annealed mixture with: the four dNTPs, each dNTP having approximately the same concentration as the others; the four ddNTPs, said ddNTPs being present in a concentration ratio selected from the group consisting of:
   (a) [ddNTP₄] > [ddNTP₃] > [ddNTP₂] > [ddNTP₁];
   (b) [ddNTP₄] > [ddNTP₃] > [ddNTP₁] > [ddNTP₂];
   (c) [ddNTP₄] > [ddNTP₂] > [ddNTP₁] > [ddNTP₃];
   (d) [ddNTP₄] > [ddNTP₂] > [ddNTP₃] > [ddNTP₁];
   (e) [ddNTP₃] > [ddNTP₄] > [ddNTP₂] > [ddNTP₁];
   (f) [ddNTP₃] > [ddNTP₄] > [ddNTP₁] > [ddNTP₂];
   (g) [ddNTP₃] > [ddNTP₁] > [ddNTP₄] > [ddNTP₂];
   (h) [ddNTP₃] > [ddNTP₁] > [ddNTP₂] > [ddNTP₄];
   (i) [ddNTP₂] > [ddNTP₄] > [ddNTP₃] > [ddNTP₁];
   (j) [ddNTP₂] > [ddNTP₄] > [ddNTP₁] > [ddNTP₃];
   (k) [ddNTP₂] > [ddNTP₁] > [ddNTP₄] > [ddNTP₃]; and
   (l) [ddNTP₂] > [ddNTP₁] > [ddNTP₃] > [ddNTP₄],
   where the numerical subscripts 1-4, inclusive, represent the same four bases of step (c) and where the concentration of the fourth position ddNTP is substantially zero; and a second DNA polymerase, under conditions in which the second DNA polymerase causes the production of a second series of at least three different DNA products, each product having the same labeled primer at a first terminus, and a ddNTP at a second terminus;
(j) separating the second series of different DNA products from the template;
(k) separating the second series of different DNA products from each other according to molecular weight to form a second arrangement of at least three different signals, each signal being specific to the concentration of each of the ddNTPs;
(l) obtaining a second data set nucleotide sequence by determining the position of each of the four ddNTPs based upon the location of the signals in the second arrangement; and
(m) comparing the first data set with the second data set to determine the nucleotide sequence of said DNA strand.
The second data set is complementary to the first data set. The difference in results is derived from the use of different concentrations of ddNTPs for the second data set relative to the concentrations of ddNTPs utilized in the first data set. Because different concentrations of ddNTPs are utilized in the second set, the intensity of the signals corresponding to these ddNTPs will be different than the intensity of the signals corresponding to the ddNTP concentrations utilized for the first data set. Because of this, both data sets will represent substantially the same nucleotide sequence, but the nucleotides in each sequence will be represented by different signals, e.g., peak heights, based upon the different ddNTP concentrations in each data set. Therefore, in the case of, for example: (a) close calls, whereby normal variations in the, e.g., peak intensities, result in artificially higher or lower peak heights; or (b) artifacts due to, e.g., shoulder peaks, the corresponding, but different, signal intensities allow for rapid and efficient discrimination of the signals such that an accurate sequence can be derived.

In the foregoing protocol, steps (g) through (l), inclusive, can be run in series or simultaneously with steps (a) through (e), inclusive. It is preferred that these steps be run simultaneously with each other for purposes of conservation of time of analysis of the DNA template strand. Additionally, the process disclosed herein is not limited to only two data set determinations. Stated again, multiple data set determinations can be made such that each consecutive data set determination utilizes a different concentration ratio of ddNTPs than the previous data set determination(s). Preferably the first and second DNA polymerases are the same polymerase, and, preferably, the first and second labels are the same label; in the case of, e.g., fluorescent labels, the first and second labels can be different labels having substantially the same emission wavelengths and excitation wavelengths. The intent is that the detected signal for the data sets be the same such that comparisons can be made between the data sets.

In a particularly preferred embodiment, the second data set is determined using the chain terminators in a concentration ratio selected from the group consisting of:
(a) [ddNTP₃] > [ddNTP₄] > [ddNTP₂] > [ddNTP₁];
(b) [ddNTP₃] > [ddNTP₄] > [ddNTP₁] > [ddNTP₂];
(c) [ddNTP₂] > [ddNTP₄] > [ddNTP₃] > [ddNTP₁]; and
(d) [ddNTP₂] > [ddNTP₄] > [ddNTP₁] > [ddNTP₃],
whereby for the second data set determination, the concentration of the ddNTP occupying the fourth position is substantially zero, i.e., in groups (a) and (c) the concentration of ddNTP₁ is substantially zero, in group (b) the concentration of ddNTP₂ is substantially zero, and in group (d) the concentration of ddNTP₃ is substantially zero.

In its most preferred embodiment, the concentration values for the first position, second position, third position and fourth position ddNTPs for the first data set are substantially equivalent to the concentration values for the first position, second position, third position and fourth position ddNTPs of the second data set, and the concentration ratio for the second data set determination is selected from the group consisting of:
(a) [ddNTP₃] > [ddNTP₄] > [ddNTP₂] > [ddNTP₁]; and
(b) [ddNTP₃] > [ddNTP₄] > [ddNTP₁] > [ddNTP₂].
For example, in its most preferred embodiment, if the concentration ratios of the ddNTPs for the first data set determination are, e.g., 4:2:1:0, then the concentration ratios of the ddNTPs for the second data set nucleotide sequence are 4:2:1:0, irrespective of the ddNTPs occupying the first position, second position, third position or fourth position for the second data set determination. A most preferred concentration ratio for both data sets is 4:1.7:0.7:0.

Preferably, the ratio of any one of the dNTPs to total ddNTPs in each data set determination is at least about 50:1 (dNTP:ddNTPs). More preferably, this ratio is between about 50:1 and about 500:1, with a most preferred ratio of about 300:1.

Within each data set, it is preferred that the concentration differences between the first and second positions be between about 3:1 and about 2:1, most preferably about 2.4:1, and between the second and third positions, between about 3:1 and about 2:1, most preferably about 2.4:1. In considering an appropriate concentration difference range, a principal criteria is the performance of the catalytic enzyme, i.e., if the discrimination in incorporation of ddNTPs over dNTPs is poor, then the concentration differences should be increased greater than about 3:1 for any consecutive positions, or, e.g., 9:3:1:0. This provides a greater dynamic range between, e.g., the peak heights. In the majority of situations, a concentration difference of about 2.4:1 between consecutive positions is acceptable, e.g., 4:1.7:0.7:0 or, e.g., 8:3.3:1.4:0.35 (note that the fourth position concentration is substantially zero, i.e., no greater than about 25% of the value of the concentration ratio of the third position).

It is to be understood that the actual concentrations used may be different than those disclosed above without departing from the spirit, scope and intent of the invention disclosed herein. Additionally, to the extent that polymerases are available or become available which discriminate against only one or some of the ddNTPs in the incorporation thereof into a growing primer strand, then the disclosure herein includes as a solution to this problem the use of an empirical concentration of that ddNTP in order to achieve a desired result. For example, if such a polymerase does not discriminate between the incorporation of, e.g., ddATP, ddTTP, and ddCTP vis-a-vis dATP, dTTP, and dCTP, respectively, but does discriminate between the incorporation of, e.g., ddGTP vis-a-vis d GTP, then a greater concentration of ddGTP, relative to the remaining ddNTPs, would have to be utilized, irrespective of the order of the ddNTP concentration ratios, in order to obtain the desired result of various achieving different signals based upon ddNTP concentration ratios.

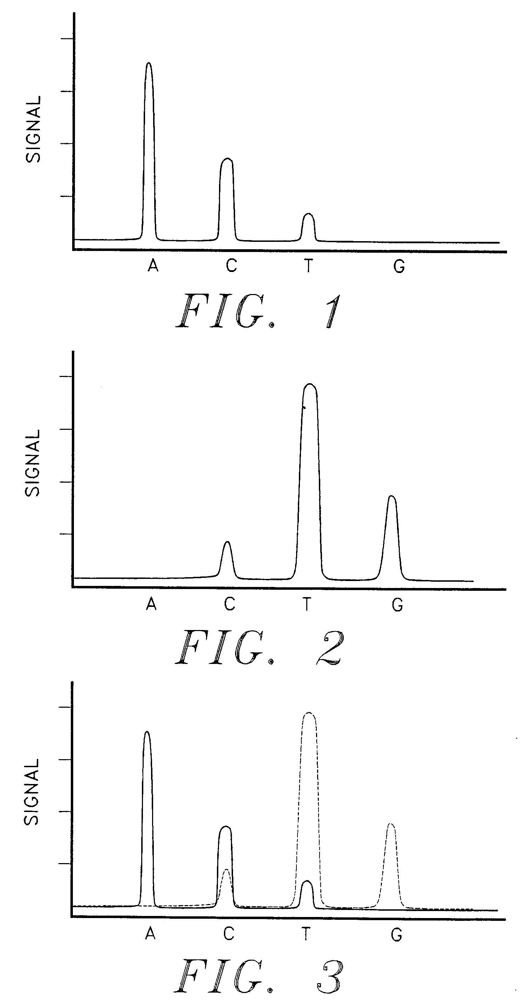

Reference is now made to FIGs. 1-3 inclusive, which are idealized electropherogram representations of the sequence ATCG, derived in accordance with the foregoing optimization protocol. In FIG. 1, the concentration ratio of ddATP:ddTTP:ddCTP:ddGTP is 4:2:1:0. Accordingly, the peak height for A is nearly twice as high as that for T, and nearly four times the height of C. For base G, for which no ddGTP is utilized in the first data set, the electropherogram evidences this by a "gap" or space. In FIG. 2, the concentration ratios are the same, but the ddNTPs represented by those ratios are different, i.e., the concentration ratio of ddTTP:ddGTP:ddCTP:ddATP is 4:2:1:0. Thus, in the second data set, the peak height for T is nearly twice the height for that of C, and nearly twice as high as G, with A being represented by a space. By comparing these results, an accurate and correct call would be made, because the second data set, in essence, validates the results obtained by the first data set. I.e., if there was a doubt as to the correctness of an initial call for, e.g., the third position (due to, e.g., a variation in the peak height which resulted in an artificial increase in this height, thus resulting in the possible assignment of a G to this position such that the putative sequence would be AGGC), then the second data set, based upon an increase concentration for the ddTTP chain terminator, would allow for proper assignment of this position on the sequence because it would be evident that the highest peak position in the second data set must be attributed to the ddNTP having the highest concentration in that set, i.e. base T.

By having the concentration of the ddNTP occupying the fourth position of the concentration ratio be substantially zero, the dynamic range of the peak heights for the other three ddNTPs increases, because there are only three peak heights between the highest peak (i.e., first position ddNTP), and the lowest "peak", or, more appropriately, "gap" (i.e., the fourth position ddNTP).

### EXAMPLES

The following Examples directed to preferred embodiments of the invention disclosed herein are not intended, nor should they be construed, as limiting the disclosure, or the claims to follow.

### I. High Performance Capillary Electrophoresis Instrumentation

For the examples to follow, a High Performance Capillary Electrophoresis/Laser Induced Fluorescence ("HPCE/LIF") instrument was utilized. A preferred format for analyzing nucleotide sequences in accordance with the protocol disclosed herein is to run at least two sequence determinations simultaneously using dual capillary columns under substantially the same operating conditions. A particularly preferred automated instrument for carrying out this format is disclosed in the above-referenced copending application, which is incorporated herein by reference. It is noted that the presently disclosed process is applicable to other automated nucleotide sequencers (which are presently commercially available and which may become available), as well as non-automated (i.e. manual) methods of nucleotide sequencing, which rely upon a dideoxy chain terminator protocol. Accordingly, while the above-referenced instrument represents a preferred format for applying the presently disclosed protocol, the disclosed protocol for optimizing nucleotide sequence determination is not to be limited thereto.

Briefly, for the HPCE/LIF format, a 2mm length of the protective polyimide coating was removed from the surface of the fused silica capillary using a thermal wire stripper (Western Electric Products Co., San Clemente, California, Model G) equipped with blade elements. This section of the capillary was supported between two compression fittings. The ends of the separation capillary were dipped in 5ml vials containing approximately 4.7ml of a running buffer solution (to be described, infra.) Connection to a high voltage power supply (for example, a Bertan Associates, Inc., Model 205A-30R power supply) was provided by paliney wires submersed in each of the buffer reservoirs. The high voltage power supply was operated in the negative polarity configuration with the outlet end of the capillary maintained at ground potential. The current passing through the gel-filed capillaries (to be described, infra) was measured as a potential drop across a 1-KΩ resistor placed in the ground side of the circuit. The system was enclosed in a plexiglass container for precautionary purposes. The temperature of the separation capillary was not regulated.

Sample introduction into the instrument was accomplished by inserting the inlet end of two separation capillaries into microfuge tubes containing the sample mixture, and a short strip of paliney wire for electrical contact to high voltage. High voltage (i.e., between about 5-50 kV) was applied for about 10-15sec.

Detection of the labelled primer was accomplished by laser induced fluorescence; for the "JOE" labelled primer (infra), detection was accomplished using the 448nm or the 514.5nm line of an air-cooled argon laser; for the BODIPY™-derivative labelled primer (infra), excitation at 543.5nm and detection at 580nm was accomplished using a helium-neon laser. Fluorescent emission was collected and directed through two interference filters (Barr Associates, Westford, Mass., product no. Custom 5 Cavity; 550nm, or 580nm, 10nm band pass) and onto the photocathode of an end-on photomultiplier tube ("PMT") (Hamamatsu, San Jose, CA. product no. R2228).

### II. Preparation of gel-filled microcapillaries

A solution comprising 50mM boric acid, 150mM tris-hydroxymethyl aminomethane ("TRIS"), 2mM EDTA and 7M urea (final pH = 9.3) was prepared and used for both preparation of the gel and for the running buffer. Fused silica capillaries (Polymicro, Technologies, Inc., part no. TSP 075375) were cut to 1meter lengths and the detector windows were created as described above in Example I. The capillaries were mounted in an in-house gel loading apparatus which is capable of pouring multiple capillaries simultaneously. Solutions were driven through the capillaries by submersing the inlet end of the capillary into the solution and then applying a head pressure of air to the solution.

The capillaries were treated with 1.0N HCl for one hour, 1.0N NaOH for one hour, followed by rinsing with methanol for ten minutes. A solution comprising of methacryloxypropyltrimethoxysilane:methanol:HCl(1.0N) in a 1:1:.04 ratio was prepared and allowed to stand at room temperature for five minutes. This was followed by introduction of this solution into the capillaries for approximately 10minutes. The capillaries were then briefly rinsed with a solution comprising HPLC grade-water and methanol in a 1:1 ratio, and flushed dry by passing air therethrough. The dry capillaries were then maintained at room temperature overnight.

An acrylamide-based gel solution was utilized for the Examples. The acrylamide/crosslinker (N,N'methylbisacrylamide) solution was prepared by addition of 10ml of the running buffer solution to 0.4g of premix acrylamide (Schwarz/Mann; 29 parts acrylamide:1 part bisacrylamide) and 0.1g of PEG 4,000 (Fluka Chemika, part no. 81240) in 10ml of running buffer solution to yield a gel composition of 4%T, 3.4%C. This monomer solution was degassed with gentle stirring for a minimum of one hour. To initiate polymerization, 10µl aliquots of 10% solutions of ammonium persulphate ("APS") and N,N,N',N'-tetramethylethylenediamine ("TEMED") were added to 2ml of the previously chilled (4°C) monomer solution. The monomer solution was then quickly introduced into the capillaries and allowed to polymerize at 4°C overnight. Once polymerization was complete, care was taken to keep the capillary ends submerged in running buffer at all times in order to prevent the gel from drying out.

Alternative gel-filled microcapillaries are, of course, applicable to the present invention. For example, a gel-filled microcapillary column applicable to the presently disclosed protocol is disclosed in United States Serial No. 07/688,182 for "High Performance Gel-Containing Microcapillary Column," filed on April 19, 1991 in the name of Chai-Hui Shieh. The foregoing reference is incorporated herein by reference.

### III. Sequencing reaction protocol

DNA sequencing reactions were performed using either the "JOE"-labelled primer (Applied Biosystems, Inc., Foster City, CA., product no. 400836) or a primer labelled with a BODIPY™-derivative having an excitation wavelength of 543nm and an emission wavelength of 580nm (proprietary to Molecular Probes, Inc.). 1µg of M13mp18 DNA (Pharmacia, Piscataway, New Jersey, product no. 27-1516-01) was mixed with 0.5picomole of the aforementioned primer and 2µl of 5X reaction buffer (USB, Cleveland, Ohio, Sequenase™ polymerase kit, product no. 70775) in a total volume of 10µl. This solution was heated to 65°C, allowed to cool to 37°C, and 1µl of DTT solution and 1µl of Mn⁺⁺ buffer (from the aforementioned Sequenase™ kit) were then added thereto. Thereafter, 9µl of a prewarmed termination mix was added thereto, followed by the addition of 1-2µl of undiluted Sequenase™ polymerase. The termination mix was comprised of concentration ratio mixture of 300 parts of one dNTP to 1 part of three of the four ddNTPs. Accordingly, the termination mix was comprised of (a) 2000µM total dNTPs (500µM of each of the four dNTPs), and (b) 1.67µM of three of the four ddNTPs in a ratio of .95µM:.48µM:.24µM:0 (4:2:1:0), or 1.04µM:0.44µM:0.18µM (4:1.7:0.7:0). The reaction was incubated for 30min at 37°C after which it was placed on ice, followed by the addition of 2µl of 3.0M sodium acetate, followed by 60µl of ice-cold ethanol. The DNA was pelleted by centrifugation at 12,000RPM for 15min and rinsed once with 70% ethanol. The pellet was then dried and resuspended in 2.0µl of 80% formamide, 10mM EDTA. The sample (which can be stored at -20°C in the dark until analyzed) was heated at 95°C for 1min just prior to injection onto the capillaries.

### Example 1

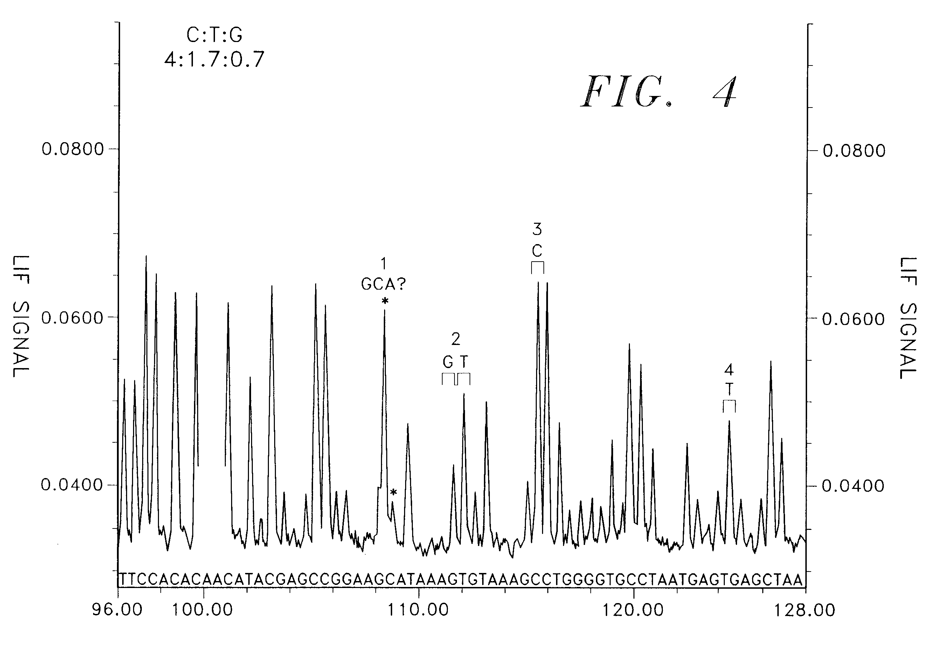

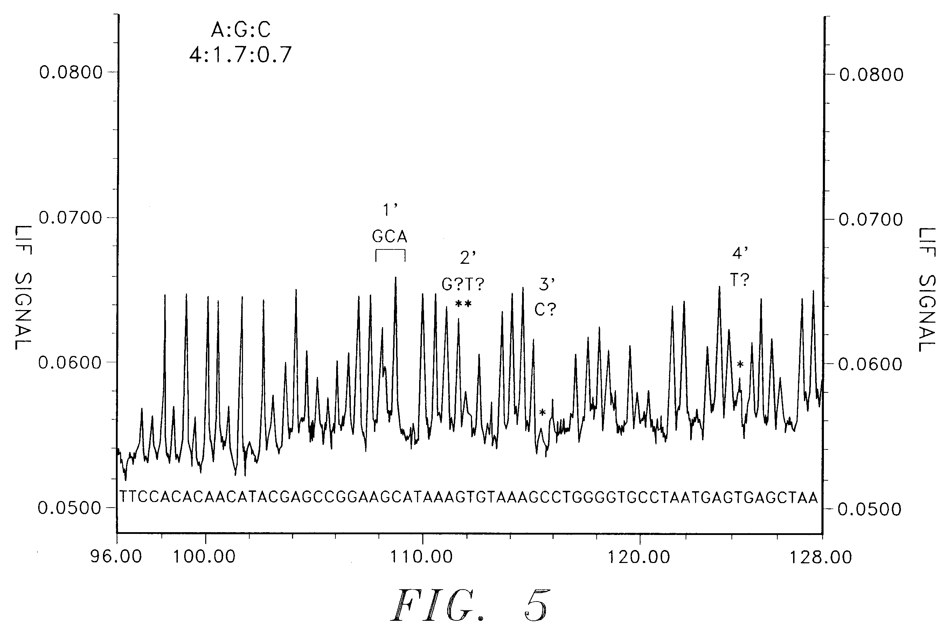

The results of the analysis of Example 1 are set forth in FIGs. 4 and 5.

Following the protocol disclosed above, the HPCE/LIF analysis of the M13mp18 bacteriophage DNA was conducted whereby the first run and the second run were conducted using concentration ratios and the dideoxynucleotides designated below: 
First Data Set (FIG. 4):
ddCTP:ddTTP:ddGTP:ddATP
4: 1.7: 0.7: 0

Second Data Set (FIG. 5):
ddATP:ddGTP:ddCTP:ddTTP
4: 1.7: 0.7: 0
This is equivalent to the following embodiment disclosed above: 
First Data Set:
[ddNTP1] > [ddNTP2] > [ddNTP3] > [ddNTP4]
Second Data Set:
[ddNTP4] > [ddNTP3] > [ddNTP1] > [ddNTP2]
Both runs were conducted simultaneously under the same operating conditions.

Referencing FIG.4, and in particular the nucleotide grouping 1, the electropherogram provides what appears to be either a single peak having a height corresponding to the highest ddNTP concentration (in this case ddCTP), or a compression artifact comprising the single peak and a smaller shoulder peak, located on the lower left-hand portion of the peak. Thus, if the call for this peak was based upon the assumption that only one peak was present, the sequence (which for this region is GCA) would evidence a deletion of a single base, i.e., the call would be CA. Thus, the entire sequence from this erroneous call forward would be off by at least one base. Those skilled in the art will immediately understand the problems associated with such a situation.

Referencing FIG. 5, nucleotide grouping 1', the use of different concentrations of the ddNTPs evidences a different electropherogram outcome. Here, the compression artifact is less pronounced, and, more critically, when compared to nucleotide grouping 1 of FIG. 4, the presence of a G base is evident. Stated again, if in the first data set, the peak was called as a single C, then in the second data set, given the fact that the concentration of ddCTP was the lowest added to the reaction mixture, one would expect a (relatively) smaller peak at this location; instead, a "medium" size peak, corresponding to the concentration of ddGTP utilized in the second data set, is present. Accordingly, by comparing nucleotide grouping 1 with 1', a more accurate analysis of the sequence is possible.

Focusing on nucleotide grouping 2' of FIG. 5, the peak to the right-hand side of the G peak could reasonably be called as C because (a) no ddTTP was present in the second data run (thus at each location for T there should be a "gap"), and (b) the peak corresponds in height to the approximate height of the ddNTP present in the lowest concentration in this set, i.e., ddCTP. However, in actuality, this base is C, as evident by the actual sequence set forth below each FIG. Thus, by relying only on the data of FIG. 5, this base would be erroneously called. When this grouping is analyzed in FIG. 4, however, it is evident that this peak should be called as T (or that an additional run or runs would be necessary to determine whether the base should be called as T or C).

Groupings 3' and 4' of FIG. 5 present additional problems. With grouping 3', the location of the peak could be construed as "background" or, more appropriately, a gap; such a gap would be expected for the ddNTP not present in the analysis, i.e., ddTTP. However, when grouping 3 of FIG. 4 is reviewed, it is evident that this position would be properly called as C. Grouping 4' provides the opposite scenario to grouping 3'. In this case, a signal peak, albeit a small peak, could be called as C, based upon the concentration of ddCTP used in the second data set; however, grouping 4 of FIG. 4 evidences that this call should be T.

The data from Example 1 indicates that by utilizing different concentrations of the ddNTPs for at least two comparative runs of the same sequence to be analyzed, a more precise determination of the correct sequence can be made, particularly with respect to close calls and compression artifacts.

### Example 2

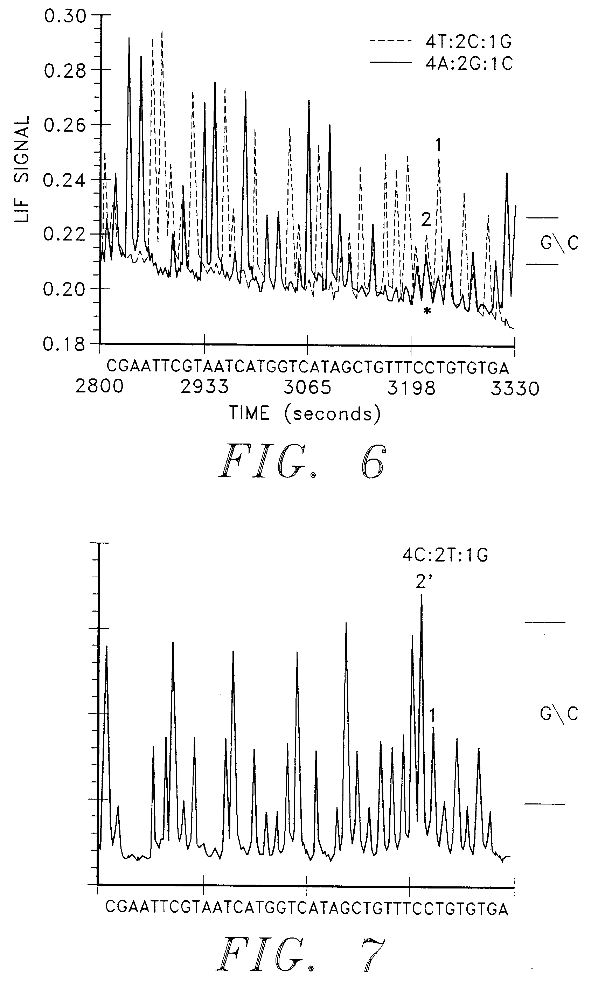

The results of the analysis of Example 2 are set forth in FIGs. 6 and 7.

Following the protocol disclosed above, the HPCE/LIF analysis of the M13mp18 bacteriophage DNA was conducted whereby the first run second run and third run were conducted using concentration ratios and the dideoxynucleotides designated below: 
First Data Set (FIG. 6, solid line):
ddATP:ddGTP:ddCTP:ddTTP
4:    2:    1:    0

Second Data Set (FIG. 6, dashed line):
ddTTP:ddCTP:ddGTP:ddATP
4:    2:    1:    0

Second' Data Set (FIG. 7):
ddCTP:ddTTP:ddGTP:ddATP
4:    2:    1:    0
This is equivalent to the following embodiment disclosed above: 
First Data Set:
[ddNTP1] > [ddNTP2] > [ddNTP3] > [ddNTP4]

Second Data Set:
[ddNTP4] > [ddNTP3] > [ddNTP2] > [ddNTP1]

Second' Data Set:
[ddNTP3] > [ddNTP4] > [ddNTP2] > [ddNTP1]
The First and Second Data Set runs were conducted simultaneously under the same operating conditions. The Second' Data Set run was conducted after the First and Second runs, under substantially the same operating conditions.

Reference is made to FIG 6. By superimposing the resulting electropherograms obtained from two data sets differing only as to the relative concentrations of the ddNTPs, an accurate sequence determination is made. Note, for example that the "gaps" of each data set (representing T for the first data set and A for the second data set), are readily verified by a comparative review of the electropherograms. Thus, for the first six bases presented on the electropherograms of FIG. 6 (CGAATT), it would be expected that the "gaps" for the first data set (T) would appear as the (relative) highest peak in the second data set, given the concentration ratios of ddTTP used in each data set (ddTTP ratio equivalent of 0 in the first data set; ddTTP ratio equivalent of 4 in the second data set). Note further that for grouping 1 of the first data set of FIG. 6, the "peak" presented could be called as C; however, this is, in actuality, an artifact, as evidenced by the (relative) high peak obtained at this position in the second data set (grouping 1'), indicating the presence of the ddNTP of the highest concentration, i.e. ddTTP.

On the right hand portion of FIG. 6 is presented the variability range of peak heights for the peaks representing G and C. At grouping 2, the lack of variability between these peak heights could lead to a reasonable call for either C or G; thus, the chances of an inaccurate call are evident. However, when compared to grouping 2' of FIG. 7, it is clear that the base must be that associated with the ddNTP present in the highest concentration in the second' data set, i.e. ddCTP. What is of note is the basis for setting forth certain of the second data set ddNTP concentrations as preferred embodiments. As previously disclosed, the second data set determination is most preferably derived from concentrations of the ddNTPs selected from the group consisting of:
(a) [ddNTP₃] > [ddNTP₄] > [ddNTP₂] > [ddNTP₁]; and
(b) [ddNTP₃] > [ddNTP₄] > [ddNTP₁] > [ddNTP₂].
As is apparent, in each of these most preferred second data set concentration ratios, the second and third ddNTP positions from the first data set are, in effect, "split" by the fourth ddNTP position from the first data set. This has at least two desirable results: first, such a relationship assists in differentiating between lowest two concentration ddNTPs utilized in the first data set; and second, by splitting these with a ddNTP not present in the first data set, the differentiation between the second and third position ddNTPs is enhanced.

As those in the art appreciate, the invention disclosed herein lends itself to automated nucleotide sequencers; when utilized in conjunction with computer software designed to accurately analyze the differences in, e.g., peak height or peak areas, the resulting analysis can be further enhanced.

While the foregoing examples have described the invention in terms of preferred embodiments and have referenced a preferred instrument for analysis, it should be understood that the invention disclosed herein is not to be limited to the specific forms or applications shown. Accordingly, equivalents and modifications which are within the purview of the skilled artisan are considered to be a part of this disclosure and the claims that follow.

## Claims

1. A process for determining the sequence of a template strand of nucleotides comprising the steps of:
(a) providing said template strand of nucleotides;
(b) annealing the template with a primer having attached thereto a first label, said first labelled primer capable of hybridizing to the template to give an annealed mixture;
(c) incubating the annealed mixture with a first incubation mixture comprising:
(1) the four dNTPs, each dNTP having approximately the same concentration as the others;
(2) ddNTPs being present in a concentration ratio [ddNTP₁] > [ddNTP₂] > [ddNTP₃] > [ddNTP₄] where each numerical subscript 1 - 4, inclusive, represents one of the four bases, A, C, G and T and where [ddNTP₄] is substantially zero; and
(3) a first polymerase,
under conditions in which the first polymerase causes the production of a first series of at least three different nucleotide products, each product having the same labelled primer at a first terminus, and a ddNTP at a second terminus;
(d) separating the first series of different nucleotide products from the template;
(e) separating the first series of different nucleotide products from each other according to molecular weight to form a first arrangement of at least three different signals, each signal being specific to the concentration of ddNTPs;
(f) providing said template;
(g) annealing the template with a primer having attached thereto a second label, said labelled primer capable of hybridizing to the template to give an annealed mixture;
(h) incubating the annealed mixture with a second incubation mixture comprising:
(1) the four dNTPs, each dNTP having approximately the same concentration as the others;
(2) ddNTPs being present in a concentration ratio selected from the group consisting of:
(a) [ddNTP₄] > [ddNTP₃] > [ddNTP₂] > [ddNTP₁];
(b) [ddNTP₄] > [ddNTP₃] > [ddNTP₁] > [ddNTP₂];
(c) [ddNTP₄] > [ddNTP₂] > [ddNTP₁] > [ddNTP₃];
(d) [ddNTP₄] > [ddNTP₂] > [ddNTP₃] > [ddNTP₁];
(e) [ddNTP₃] > [ddNTP₄] > [ddNTP₂] > [ddNTP₁];
(f) [ddNTP₃] > [ddNTP₄] > [ddNTP₁] > [ddNTP₂];
(g) [ddNTP₃] > [ddNTP₁] > [ddNTP₄] > [ddNTP₂];
(h) [ddNTP₃] > [ddNTP₁] > [ddNTP₂] > [ddNTP₄];
(i) [ddNTP₂] > [ddNTP₄] > [ddNTP₃] > [ddNTP₁];
(j) [ddNTP₂] > [ddNTP₄] > [ddNTP₁] > [ddNTP₃];
(k) [ddNTP₂] > [ddNTP₁] > [ddNTP₄] > [ddNTP₃]; and
(l) [ddNTP₂] > [ddNTP₁] > [ddNTP₃] > [ddNTP₄];
where the numerical subscripts 1 - 4, inclusive, represent the same four bases of step (c) and where the concentration of the fourth position ddNTP is substantially zero;
(3) and a second polymerase,
under conditions in which the second polymerase causes the production of a second series of at least three different nucleotide products, each product having the same labelled primer at a first terminus, and a ddNTP at a second terminus;
(i) separating the second series of different nucleotide products from the template; and
(j) separating the second series of different nucleotide products from each other according to molecular weight to form a second arrangement of at least three different signals, each signal being specific to the concentration of each of the ddNTPs,
wherein the nucleotide sequence is determined based upon the first arrangement of at least three different signals and the second arrangement of at least three different signals.

2. The process of claim 1,
wherein a first data set nucleotide sequence is determined from the position of each of said signals of said first arrangement, and a second data set nucleotide sequence is determined from the position of each of said signals of said second arrangement.

3. The process of claim 1 or 2,
wherein said first and said second data sets are compared with each other.

4. The process of any of claims 1 to 3,
wherein said first polymerase and said second polymerase are substantially the same.

5. The process of any of claims 1 to 4,
wherein said first label and said second label are substantially the same label.

6. The process of any of claims 1 to 5,
wherein the ratio of one of the dNTPs to ddNTPs in the first incubation mixture is greater than about 50 : 1.

7. The process of any of claims 1 to 6,
wherein the ratio of one of the dNTPs to ddNTPs in the second incubation mixture is greater than about 50 : 1.

8. The process of any of claims 1 to 7,
wherein the concentration ratio of ddNTP₁ to ddNTP₂ in the first incubation mixture is between about 3 : 1 and about 2 : 1.

9. The process of claim 8,
wherein the concentration ratio of ddNTP₂ to ddNTP₃ in the first incubation mixture is between about 3 : 1 and about 2 : 1.

10. The process of any of claims 1 to 9,
wherein the concentration ratios of ddNTPs present in the first incubation mixture are substantially the same as the concentration ratios of ddNTPs present in the second incubation mixture.

11. The process of any of claims 1 to 10,
wherein the ratio of [ddNTP₁] : [ddNTP₂] : [ddNTP₃] in said first incubation mixture is 4 : 1.7 : 0.7.

12. The process of any of claims 1 to 11,
wherein steps (a) through (e) are conducted simultaneously with steps (f) through (j).

13. The process of any of claims 1 to 12,
**characterized** in
that step (h) is performed by incubating the annealed mixture with a second incubation mixture comprising:
(1) the four dNTPs, each dNTP having approximately the same concentration as the others; and
(2) ddNTPs being present in a concentration ratio selected from the group consisting of those designated in claim 1 as (e), (f), (i) and (j).

14. The process of claim 13,
**characterized** in
that step (h) is performed by incubating the annealed mixture with a second incubation mixture comprising
(1) the four dNTPs, each dNTP having approximately the same concentration as the others; and
(2) ddNTPs being present in a concentration ratio selected from the group consisting of those designated in claim 1 as (e) and (f).
